# EUROPEAN PATENT APPLICATION

(11) **EP 2 036 888 A1**
(43) Date of publication of application: **18.03.2009**
(21) Application number: 07384034.0
(22) Date of filing: 17.09.2007
(51) Int. Cl.: C07D 209/14, A61K 31/4045, A61P 29/00, A61P 25/00, A61P 25/28, A61P 3/04

(54) **Naphthyl-substituted sulfonamides**

(71) Applicant: Laboratorios del Dr. Esteve S.A., 08041 Barcelona (ES)
(72) Inventor: Merce-Vidal, Ramòn, 08021 Barcelona (ES)
(74) Representative: Peters, Hajo

(57) **Abstract**

The present application relates to naphtyl substituted sulfonamides according to general formula (I) with 5-HT₆ receptor affinity, a medicament comprising a said compound, and the use of one of said compounds for the manufacture of a medicament.

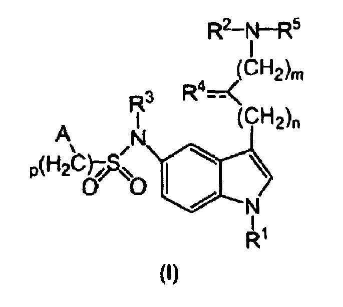

## Description

The present invention relates to naphtyl substituted sulfonamides with 5-HT₆ receptor affinity, a medicament comprising a said compound, and the use of one of said compounds for the manufacture of a medicament.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is a serotonin receptor identified by molecular cloning both in rats [F.J. Monsma, et al., Mol. Pharmacol., 1993, 43, 320; M. Ruat, et al., Biochem. Biophys. Res. Commun., 1993, 193, 268] and in humans [R. Kohen, et al., J. Neurochem., 1996, 66, 47]. Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka, et al., Ann. NY Acad. Sci., 1998, 861, 244; A. Bourson, et al., Br. J. Pharmacol. , 1998, 125, 1562; D.C. Rogers, et al., Br. J. Pharmacol. Suppl., 1999, 127, 22P; A. Bourson, et al., J. Pharmacol. Exp. Ther. , 1995, 274, 173; A.J. Sleight, et al., Behav. Brain Res. , 1996, 73, 245; T.A. Branchek, et al., nnu. Rev. Pharmacol. Toxicol. , 2000, 40, 319; C. Routledge, et al., Br. J. Pharmacol. , 2000, 130, 1606]. It has been shown that typical and atypical antipsychotic drugs for treating schizophrenia have a high affinity for 5-HT₆ receptors [B.L. Roth, et al., J. Pharmacol. Exp. Ther. , 1994, 268, 1403; C.E. Glatt, et al., Mol. Med. , 1995, 1, 398; F.J. Mosma, et al., Mol. Pharmacol. , 1993, 43, 320; T. Shinkai, et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst, et al., Br. J. Pharmacol. , 2000, 130, 1597; C. Gérard, et al., Brain Research , 1997, 746, 207; M.R. Pranzatelli, Drugs of Today , 1997, 33, 379]. Moreover, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases.

Thus, the object of the present invention was to provide medicaments that comprise compounds with 5-HT₆ receptor affinity and which are suitable for the prophylaxis and/or treatment of food-ingestion related disorders.

In particular, it was an object of the present invention to provide a medicament suitable for the prophylaxis and/or treatment of obesity/food ingestion disorders, which preferably does not show the undesired side effects of the conventional medicaments, or at least less frequent and/or less pronounced.

It has been found that the compounds of general formula (I) given below show affinity for the 5-HT₆-receptor. These compounds are therefore also suitable for the manufacture of a medicament for the prophylaxis and/or treatment of food ingestion (food intake) disorders, particularly for the regulation of appetite, for the maintenance, increase or reduction of body weight, for the prophylaxis and/or treatment of obesity, bulimia, anorexia, cachexia or type II diabetes (Non-Insulin Dependent Diabetes Mellitus), preferably type II diabetes, which is caused by obesity or for the manufacture of a medicament for the prophylaxis and/or treatment of cognitive disorders.

Compounds showing similarities to the compounds according to the invention are being found in EP 1 445 252 A1, describing also compounds binding to the 5HT₆-receptor.

Said object has been achieved by providing as an active substance a naphthyl-substituted sulfonamide compound according to general formula (I) 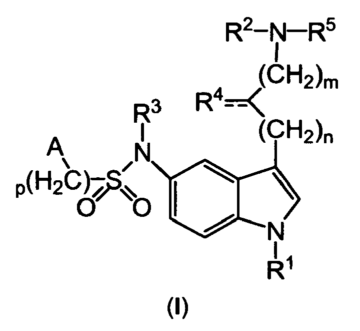 wherein
**A** represents a naphthyl radical, unsubstituted, or mono- or di-substituted with any of U, **V** or **W** selected from halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl or a benzyl radical;
**R²** represents hydrogen, C₁-C₄ alkyl; optionally at least monosubstituted alkyl-aryl; or C(O)-R⁶ with R⁶ being at least mono-substituted aryl;
**R³** represents hydrogen or C₁-C₄ alkyl;
**R⁴** represents hydrogen or C₁-C₄ alkyl;
   **or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring, optionally condensed with an un-substituted phenyl;
**n** represents 0, or 1;
**m** represents 0, 1 or 2;
**p** represents 0, 1 or 2;
^{....} represents either a single or a double bond;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In the context of this invention, alkyl radical or group is understood as meaning saturated, linear or branched hydrocarbons, which can be unsubstituted or mono- or polysubstituted. In these radicals, C₁₋₂-alkyl represents C1- or C2-alkyl, C₁₋₃-alkyl represents C1-, C2- or C3-alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkyl group - unless defined otherwise - the term substituted in the context of this invention is understood as meaning replacement of at least one hydrogen radical by F, Cl, Br, I, NH₂, SH or OH, "polysubstituted" (more than once substituted) radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. "Optionally at least monosubstituted" means either "monosubstituted", "polysubstituted" or - if the option is not fulfilled - "unsubstituted".

The term (CH₂)₃₋₆ is to be understood as meaning -CH₂-CH₂-CH₂-, -CH₂-CH₂-CH₂-CH₂-, - CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc.

A heterocyclyl radical or group is understood as meaning heterocyclic ring systems, of one, two or three saturated or unsaturated rings of which at least one contains one or more heteroatoms from the group consisting of nitrogen, oxygen and/or sulfur in the ring and can also be mono- or polysubstituted. Examples to be mentioned from the group of heteroaryls are furan, benzofuran, thiophene, benzothiophene, pyrrole, pyrrolidine, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In connection with heterocyclyl, substituted is understood - unless defined otherwise - as meaning substitution of the ring-system of the heterocyclyl by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl-group; a saturated or unsaturated, linear or branched, substituted or unsubstituted -C(O)-O-C₁₋₆-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl. "Optionally at least monosubstituted" means either "monosubstituted", "polysubstituted" or - if the option is not fulfilled - "unsubstituted".

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids and in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Any compound that is a prodrug of a compound of formulas (I), (II), (III) or (IV) is within the scope of the invention. The term "prodrug" is used in its broadest sense and encompasses those Derivatives that are converted in vivo to the compounds of the invention. Such Derivatives would readily occur to those skilled in the art, and include, depending on the functional groups present in the molecule and without limitation, the following Derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

The compounds of formulas (I), (II), (III) or (IV) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), (II), (III) or (IV), or of its salts, solvates or prodrugs.

In a preferred embodiment of the naphthyl-substituted sulfonamide compounds according to the invention according to formula (I) the compounds are compounds according to formula (la) 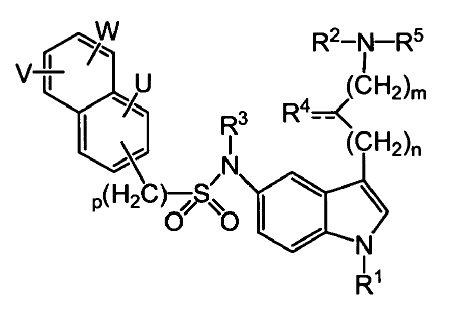 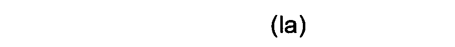 wherein
**U**, **V** and **W** independently from one another represent hydrogen; halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl or a benzyl radical;
**R²** represents hydrogen, C₁-C₄ alkyl; optionally at least monosubstituted alkyl-aryl; or C(O)-R⁶ with R⁶ being at least mono-substituted aryl;
**R³** represents hydrogen or C₁-C₄ alkyl;
**R⁴** represents hydrogen or C₁-C₄ alkyl;
   **or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring, optionally condensed with an un-substituted phenyl;
**n** represents 0, or 1;
**m** represents 0, 1 or 2.

In another preferred embodiment of the naphthyl-substituted sulfonamide compounds according to the invention according to formula (I) the compounds are compounds according to general formula (II) 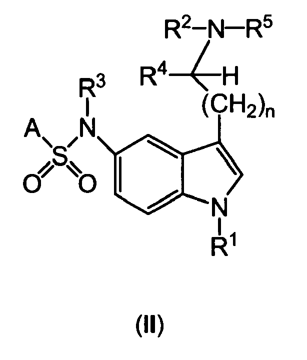 wherein
**A** represents a naphthyl radical, unsubstituted, or mono- or di-substituted with any of U, **V** or **W** selected from halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
   **or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (II) the compounds are compounds according to general formula (IIa) 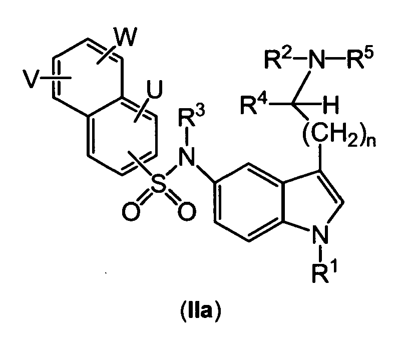 wherein
**U**, **V** and **W** independently from one another represent hydrogen; halogen; C₁-C₄ alkyl; or NR⁶R⁷ or OR⁶ with R⁶ and R⁷ independently from one another being selected from H or C₁-C₄ alkyl;
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
   **or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring;
**n** represents 0 or 1;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (II) the compounds are compounds according to general formula (IIb) 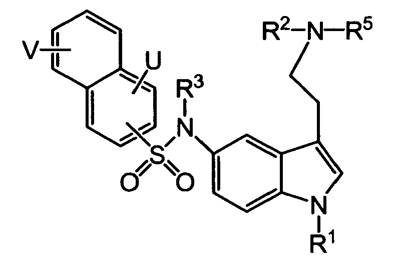 , wherein
one of **U** and **V** represents hydrogen; halogen; C₁-C₄ alkyl; or NR⁶R⁷ or OR⁶ with R⁶ and R⁷ independently from one another being selected from H or C₁-C₄ alkyl, while the other represents hydrogen;
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
   **or**
**R²** and **R⁵** together with the connecting nitrogen are forming an unsubstituted pyrrolidine.
   In another preferred embodiment of the naphthyl-substituted sulfonamide compounds according to the invention according to formula (II)
   one of U and V represents hydrogen; F, Cl; methyl; or N(CH₃)₂ or OR⁶ with R⁶ being selected from H or C₁-C₄ alkyl, while the other represents hydrogen;
**R¹** represents hydrogen, methyl or ethyl; preferably represents hydrogen or methyl; **and/or**
**R³** represents hydrogen, methyl, or ethyl; preferably represents hydrogen or methyl; **and/or**
**R²** represents hydrogen, methyl, ethyl or propyl; preferably represents methyl;
**R⁵** represents hydrogen, methyl or ethyl; preferably represents methyl;
   **or**
**R²** and **R⁵** together with the connecting nitrogen are forming an unsubstituted pyrrolidine.

In another preferred embodiment of the naphthyl-substituted sulfonamide compounds according to the invention according to formula (II) the compounds are selected from
- 6-chloro-N-(3-(2-(dimethylamino)ethyl)-1H-indol-5-yl)naphthalene-2-sulfonamide;
- 5-(dimethylamino)-N-(3-(2-(dimethy)amino)ethyl)-1H-indol-5-yl)naphthalene-1-sulfonamide;
- N-(3-(2-(dimethylamino)ethyl)-1H-indol-5-yl)-4-methylnaphthalene-1-sulfonamide;
- 7-chloro-N-(3-(2-(dimethylamino)ethyl)-1H-indol-5-yl)naphthalene-2-sulfonamide;
- 6-(dimethylamino)-N-(3-(2-(dimethylamino)ethyl)-1H-indol-5-yl)naphthalene-2-sulfonamide;
- 8-chloro-N-(3-(2-(dimethylamino)ethyl)-1H-indol-5-yl)naphthalene-1-sulfonamide;
- N-(3-(2-(dimethylamino)ethyl)-1H-indol-5-yl)-4-fluoronaphthalene-1-sulfonamide
- 5-chloro-N-(3-(2-(dimethylamino)ethyl)-1-methyl-1H-indol-5-yl)naphthalene-2-sulfonamide;
- N-(1-methyl-3-(2-(pyrrolidin-1-yl)ethyl)-1H-indol-5-yl)naphthalene-1-sulfonamide
- N-(3-(2-(dimethylamino)ethyl)-1H-indol-5-yl)-N-methylnaphthalene-1-sulfonamide;
- N-(3-(2-(dimethylamino)ethyl)-1H-indol-5-yl)-N-methylnaphthalene-2-sulfonamide;
- 5-chloro-N-(3-(2-(dimethylamino)ethyl)-1H-indol-5-yl)-N-methylnaphthalene-1-sulfonamide;
- 5-chloro-N-(3-(2-(dimethylamino)ethyl)-1H-indol-5-yl)-N-methylnaphthalene-2-sulfonamide;
- N-(3-(2-(dimethylamino)ethyl)-1H-indol-5-yl)-5-methoxynaphthalene-1 - sulfonamide;
- N-(3-(2-(dimethylamino)ethyl)-1H-indol-5-yl)-5-hydroxynaphthalene-1-sulfonamide;
- N-(3-(2-(dimethylamino)ethyl)-1H-indol-5-yl)-7-methoxynaphthalene-2-sulfonamide;
- N-(3-(2-(dimethylamino)ethyl)-1H-indol-5-yl)-7-hydroxynaphthalene-2-sulfonamide;
- N-(3-(2-(dimethylamino)ethyl)-1H-indol-5-yl)-8-methoxynaphthalene-1-sulfonamide;
- N-(3-(2-(dimethylamino)ethyl)-1H-indol-5-yl)-8-hydroxynaphthalene-1-sulfonamide; or
- N-(3-(2-(dimethylamino)ethyl)-1H-indol-5-yl)-4-methoxynaphthalene-1-sulfonamide;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (I), the compounds are compounds according to formula (III) 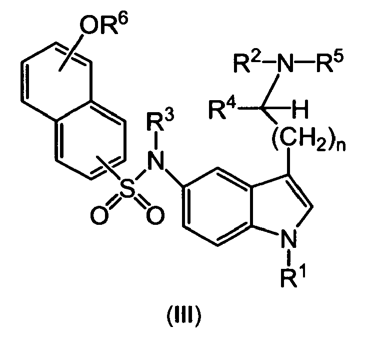 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
**or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring;
**R⁶** represents hydrogen or C₁-C₄ alkyl;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (III) the compounds are compounds according to formula (IIIa) 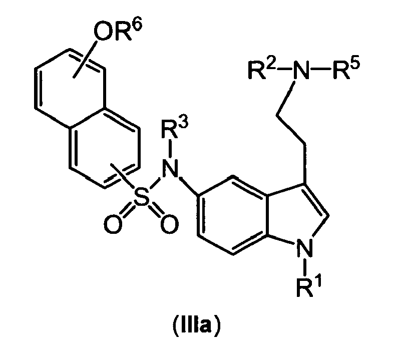 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
   **or**
**R²** and **R⁵** together with the connecting nitrogen are forming an unsubstituted pyrrolidine;
**R⁶** represents hydrogen or C₁-C₄ alkyl.
In another preferred embodiment of the naphthyl-substituted sulfonamide compounds according to the invention according to formula (III)
**R¹** represents hydrogen, methyl or ethyl; preferably represents hydrogen or methyl; **and/or**
**R³** represents hydrogen, methyl, or ethyl; preferably represents hydrogen or methyl; **and/or**
**R⁶** represents hydrogen, methyl, or ethyl; preferably represents hydrogen or methyl; and/or
**R²** represents hydrogen, methyl, ethyl or propyl; preferably represents methyl;
**R⁵** represents hydrogen, methyl or ethyl; preferably represents methyl;
   **or**
**R²** and **R⁵** together with the connecting nitrogen are forming an unsubstituted pyrrolidine.
In another preferred embodiment of the naphthyl-substituted sulfonamide compounds according to the invention according to formula (III) the compounds are selected from
- N-(3-(2-(dimethylamino)ethyl)-1H-indol-5-yl)-5-methoxynaphthalene-1-sulfonamide;
- N-(3-(2-(dimethylamino)ethyl)-1H-indol-5-yl)-5-hydroxynaphthalene-1-sulfonamide;
- N-(3-(2-(dimethylamino)ethyl)-1H-indol-5-yl)-7-methoxynaphthalene-2-sulfonamide;
- N-(3-(2-(dimethylamino)ethyl)-1H-indol-5-yl)-7-hydroxynaphthalene-2-sulfonamide;
- N-(3-(2-(dimethylamino)ethyl)-1H-indol-5-yl)-8-methoxynaphthalene-1-sulfonamide;
- N-(3-(2-(dimethylamino)ethyl)-1H-indol-5-yl)-8-hydroxynaphthalene-1-sulfonamide; or
- N-(3-(2-(dimethylamino)ethyl)-1H-indol-5-yl)-4-methoxynaphthalene-1-sulfonamide;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (I), the compounds are compounds according to formula (IV) 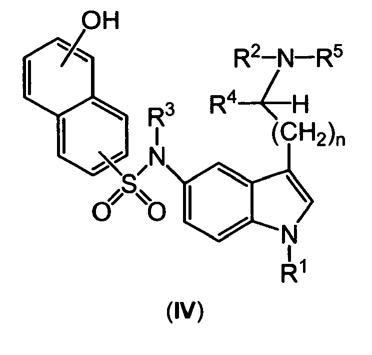 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
**or**
**R²** and **R⁴** together with the connecting atoms are forming an optionally monosubstituted, optionally monounsaturated 5- or 6-membered heterocyclic ring;
**R⁵** represents hydrogen or C₁-C₄ alkyl
   **or**
**R²** and **R⁵** together with the connecting nitrogen are forming an optionally monosubstituted 5- or 6-membered heterocyclic ring;
**n** represents 0 or 1;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In another preferred embodiment of the naphthyl-substituted sulfonamide compounds according to the invention according to formula (IV), the compounds are compounds according to formula (IVa) 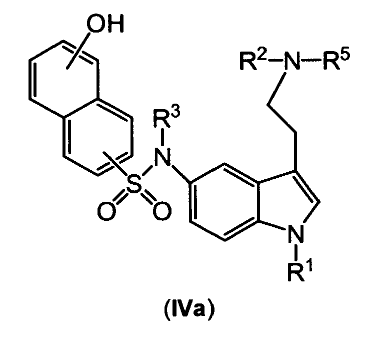 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
   **or**
**R²** and **R⁵** together with the connecting nitrogen are forming an unsubstituted pyrrolidine.

In another preferred embodiment of the naphthyl-substituted sulfonamide compounds according to the invention according to formula (IV)
**R¹** represents hydrogen, methyl or ethyl; preferably represents hydrogen or methyl; **and/or**
**R³** represents hydrogen, methyl, or ethyl; preferably represents hydrogen or methyl; **and/or**
**R²** represents hydrogen, methyl, ethyl or propyl; preferably represents methyl;
**R⁵** represents hydrogen, methyl or ethyl; preferably represents methyl;
   **or**
**R²** and **R⁵** together with the connecting nitrogen are forming an unsubstituted pyrrolidine.

In another preferred embodiment of the naphthyl-substituted sulfonamide compounds according to the invention according to formula (IV) the compounds are selected from
- N-(3-(2-(dimethylamino)ethyl)-1H-indol-5-yl)-5-hydroxynaphthalene-1-sulfonamide;
- N-(3-(2-(dimethylamino)ethyl)-1H-indol-5-yl)-7-hydroxynaphthalene-2-sulfonamide; or
- N-(3-(2-(dimethylamino)ethyl)-1H-indol-5-yl)-8-hydroxynaphthalene-1-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

The compounds according to the invention according to general formula (I) or general formula (la) respectively are prepared according to reaction scheme I. The starting material for the respective synthesis according to all the reaction schemes shown below are either commercially available or can be prepared according to standard methods. 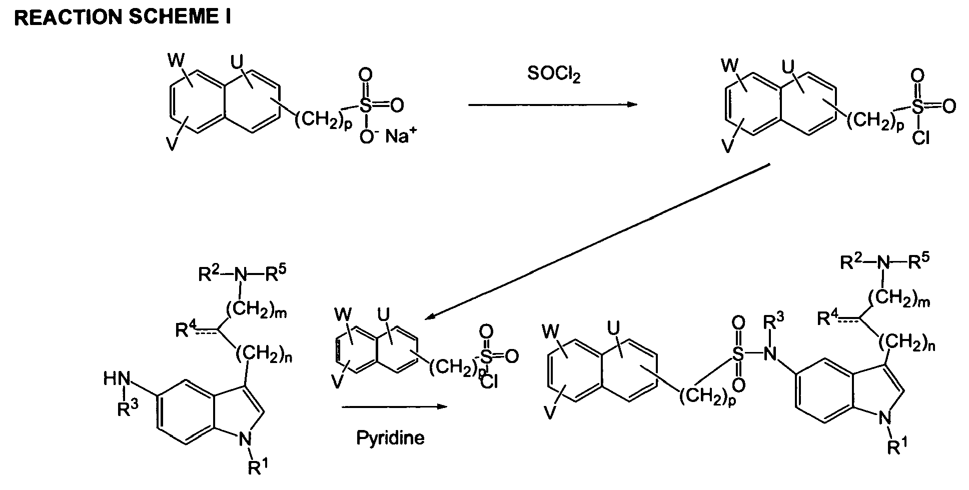 The compounds according to the invention according to general formula (II) or general formula (IIa) respectively are prepared according to reaction scheme II. 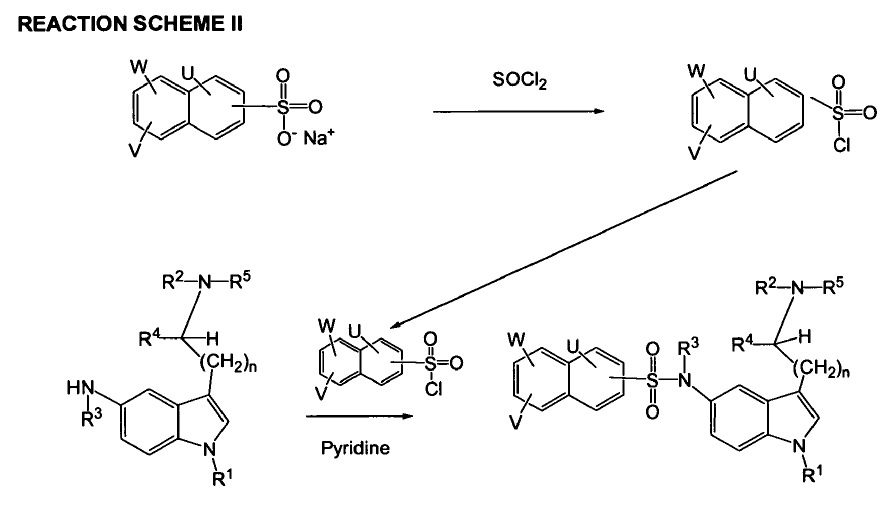 The compounds according to the invention according to general formula (III) or (IV) respectively are prepared according to reaction scheme III. 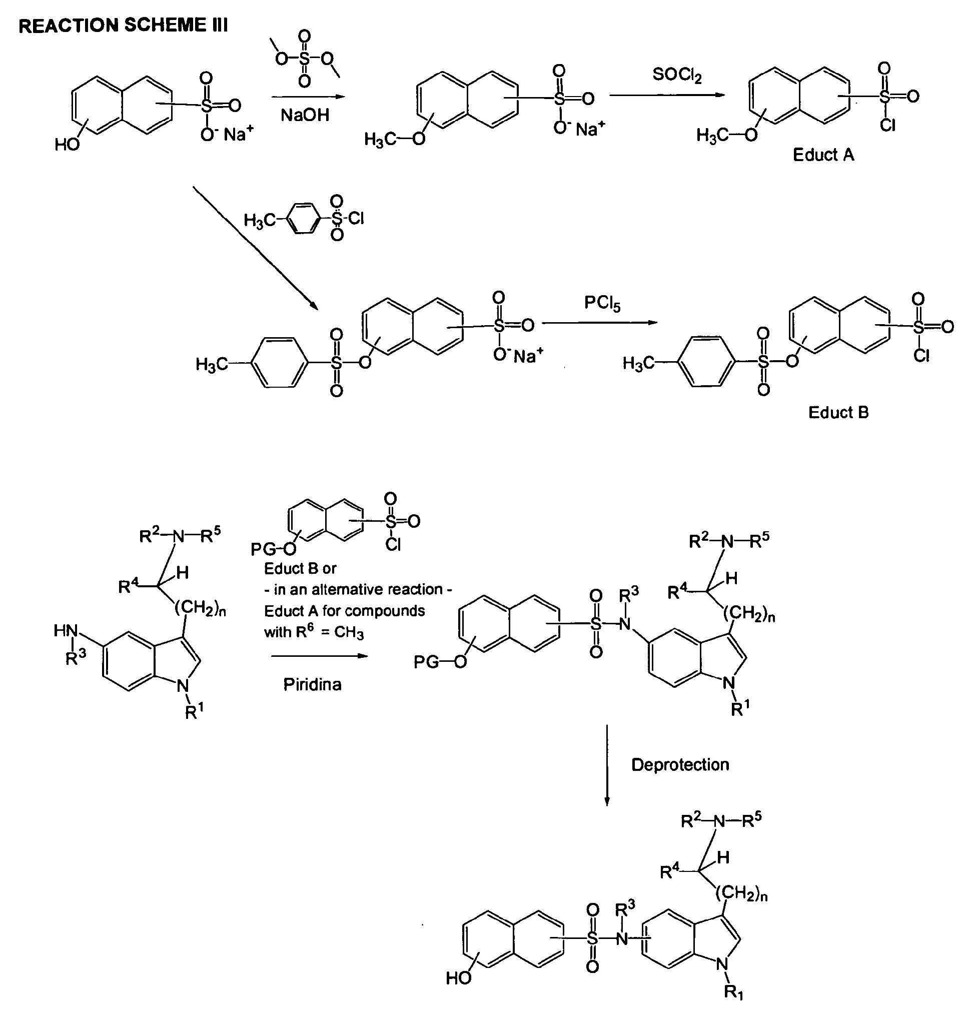 In general in addition to the information provided by the general reaction schemes and the examples set out below, the preparation of the pyrrolidine compounds was done according to D.C. Cole et al., J.Med.Chem. (2005); 48, 353-356.

Besides this process the invention further provides a further aspect in a process for the preparation of salts of compounds of general formula (I), (II), (III) or (IV), wherein at least one compound of general formula (I), (II), (III) or (IV) is reacted with an inorganic and/or organic acid, preferably in the presence of a suitable reaction medium. Suitable reaction media are the ones given above. Suitable inorganic acid are for example hydrochloric acid, hydrobromic acid, phosphoric acid, sulphuric acid, nitric acid. Suitable organic acids are e.g. citric acid, maleic acid, furmaric acid, tartaric acid or derivatives thereof, such as p-toluenesulfonic acid, methanesulfonic acid or camphersulfonic acid.

In yet a further aspect the present invention also provides a process for the preparation of salts of compounds of general formula (I), (II), (III) or (IV) wherein at least one compound of general formula (I), (II), (III), or (IV) having at least one acidic group is reacted with one or more suitable bases, preferably in the presence of suitable reaction medium. Suitable bases are e.g. hydroxides. Carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or linear C₁₋₄ alkyl radical.

Solvates, preferably hydrates, of the compounds of general formula (I), (II), (III) or (IV) or corresponding stereoisomers, or corresponding salts may also be obtained by standard procedures known to those skilled in the art.

If the compounds of general formula (I), (II), (III) or (IV) are obtained in form of a mixture of stereoisomers, particularly enantiomers or diastereomers, said mixtures may be separated by standard procedures known to those skilled in the art, e.g. chromatographic methods of crystallization with chiral reagents.

The purification and isolation of the compounds of general formula (I), (II), (III) or (IV) or a corresponding stereoisomer, or a corresponding salt, or corresponding solvate respectively, if required may be carried out by conventional methods known to those skilled in the art, e.g. chromatographic methods or recrystallization.

The compounds of general formula (I), (II), (III) or (IV) their stereoisomers or the respective salts or solvates are toxicologically acceptable and are therefore suitable as pharmaceutical active substances for the preparation of medicaments.

The present invention therefore also provides for a medicament comprising at least one compound of general formula (I), (Ia), (II), (IIa), (IIb), (III), (IIIa), (IV) or (IVa), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, and optionally one or more pharmaceutically acceptable adjuvants.

Furthermore, the present invention also provides for a pharmaceutical composition comprising at least one compound of general formula (I), (II), (III) or (IV) optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

Preferably the medicament is suitable for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

Preferably the medicament is also suitable for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition; especially

for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome.

The present invention also provides for the use of at least one compound of general formula (I), (II), (III) or (IV), optionally in form of one of its stereoisomers, preferably enantiomers or diastereomers, its racemate or in form of a mixture of at least two of its stereoisomers, preferably enantiomers or diastereomers, in any mixing ratio, or a physiologically acceptable salt thereof, or a solvate, respectively, for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

The medicament may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the compound of general formula (I), (II), (III) or (IV) optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substance to be administered during one or several intakes.

The following examples are provided to illustrate the claimed invention and are not meant in any way to limit it:

### Examples

### Example 1:

6-chloro-N-(3-(2-(dimethylamino)ethyl)-1H-indol-5-yl)naphthalene-2-sulfonamide

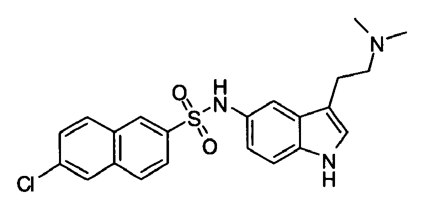

To a cooled solution of 3-(2-(dimethylamino)ethyl)-1H-indol-5-amine (73,1 mg, 0,36 mmol) in 4 ml of dry pyridine a solution of 6-chloronaphthalene-2-sulfonyl chloride (103 mg, 0,40 mmol) in 2 mL of dry pyridine was added dropwise between -10 and 0°C degrees. The dark solution was stirred at 0°C for 1 h and at room temperature overnight. A solution of 4 mL of saturated solution of NaHCO3 and 1 mL of NH₄OH was added. Then, the solvent was removed at reduced pressure and the residue obtained was purified by flash chromatography (silicagel) with CH₂Cl₂/MeOH/NH₄OH (8:2:0,02) yielding 154 mg (49%) of 6-chloro-N-(3-(2-(dimethylamino)ethyl)-1H-indol-5-yl)naphthalene-2-sulfonamide as a solid
Melting point: 170-172°C
1 H NMR (300 MHz, DMSO-*d*₆) δ ppm 2.03 (s, 6 H) 2.19 (m, 2 H) 2.56 (m, 2 H) 6.79 (dd, *J*=8.5, 2.0 Hz, 1 H) 7.01 (d, *J*=2.0 Hz, 1 H) 7.05 (d, *J*=2.0 Hz, 1 H) 7.13 (d, *J*=8.50 Hz, 1 H) 7.60 (dd, *J*=8.8, 2.1 Hz, 1 H) 7.79 (dd, *J*=8.8, 1.6 Hz, 1 H) 8.04 (d, *J*=8.8 Hz, 1 H) 8.09 (d, *J*=8.8 Hz, 1 H) 8.12 (d, *J*=2.1 Hz, 1 H) 8.29 (d, *J*=1.6 Hz, 1 H) 9.86 (br. s., 1 H) 10.73 (d, *J*=2.0 Hz, 2 H)

The following compounds may be prepared by a similar procedure from the corresponding aminoindoles:

### Example 2:

5-(dimethylamino)-N-(3-(2-(dimethylaminolethyl)-1H-indol-5-yl)nanhthalene-1-sulfonamide 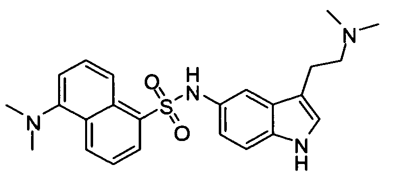 Melting point: 93-96°C

1 H NMR (300 MHz, DMSO-*d*₆) δ ppm 2.09 (s, 6 H) 2.23 (m, 2 H) 2.54 (m, 2 H) 2.78 (s, 6 H) 6.72 (dd, *J*=8.6, 2.1 Hz, 1 H) 6.94 (d, *J*=2.1 Hz, 1 H) 7.02 (d, *J*=2.1 Hz, 1 H) 7.07 (d, J=8.6 Hz, 1 H) 7.23 (d, J=7.0 Hz, 1 H) 7.47 (dd, *J*=8.5, 7.3 Hz, 1 H) 7.60 (dd, *J*=8.6, 7.6 Hz, 1 H) 8.01 (dd, *J*=7.4, 1.3 Hz, 1 H) 8.36 (d, *J*=8.4 Hz, 1 H) 8.44 (d, *J*=8.6 Hz, 1 H) 10.07 (br. s., 1 H) 10.68 (d, *J*=1.8 Hz, 1 H)

### Example 3:

N-(3-(2-(dimethylamino)ethyl)-1H-indol-5-yl)-4-methylnanhthalene-1-sulfonamide 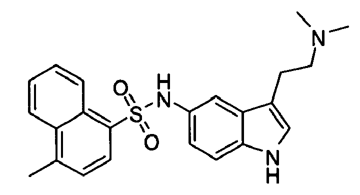 Melting point: 91-93°C

1 H NMR (300 MHz, DMSO-*d*₆) δ ppm 2.12 (s, 6 H) 2.24 (m, 2 H) 2.55 (m, 2 H) 2.63 (s, 3 H) 6.68 (dd, *J*=8.6, 2.0 Hz, 1 H) 6.95 (d, *J*=2.0 Hz, 1 H) 7.02 (d, J=2.1 Hz, 1 H) 7.05 (d, J=8.6 Hz, 1 H) 7.35 (dd, J=7.5, 1.0 Hz, 1 H) 7.61 - 7.77 (m, 2 H) 7.93 (d, J=7.5 Hz, 1 H) 8.11 (m, 1 H) 8.81 (m, 1 H) 10.07 (br. s., 1 H) 10.66 (s, 1 H)

### Example 4:

7-chloro-N-(3-(2-(dimethylamino)ethyl)-1H-indol-5-yl)naphthalene-2-sulfonamide 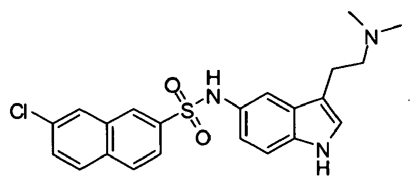 Melting point: 84-88°C

1 H NMR (300 MHz, DMSO-*d*₆) δ ppm 2.04 (s, 6 H) 2.21 (m, 2 H) 2.58 (m, 2 H) 6.79 (dd, *J*=8.6, 1.8 Hz, 1 H) 7.04 (d, *J*=1.5 Hz, 1 H) 7.05 (d, *J*=1.8 Hz, 1 H) 7.14 (d, *J*=8.6 Hz, 1 H) 7.66 (dd, *J*=8.7, 2.2 Hz, 1 H) 7.77 (dd, *J*=8.7, 1.8 Hz, 1 H) 8.01 (d, *J*=8.7 Hz, 1 H) 8.09 (d, *J*=8.7 Hz, 1 H) 8.21 (d, *J*=1.8 Hz, 1 H) 8.28 (s, 1 H) 9.89 (br. s., 1 H) 10.74 (s, 1 H)

### Example 5:

6-(dimethylamino)-N-(3-(2-(dimethylamino)ethyl)-1H-indol-5-yl)naphthalene-2-sulfonamide 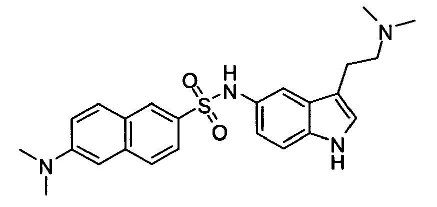 Melting point: 187-190°C

1 H NMR (300 MHz, DMSO-*d*₆) δ ppm 2.06 (s, 6 H) 2.24 (m, 2 H) 2.58 (m, 2 H) 3.00 (s, 6 H) 6.78 (dd, *J*=8.5, 2.0 Hz, 1 H) 6.89 (d, *J*=2.3 Hz, 1 H) 7.03 (d, *J*=2.2 Hz, 1 H) 7.06 (d, *J*=2.0 Hz, 1 H) 7.10 (d, *J*=8.5 Hz, 1 H) 7.23 (dd, *J*=9.1, 2.4 Hz, 1 H) 7.52 (dd, *J*=8.7, 1.8 Hz, 1 H) 7.68 (d, *J*=8.7 Hz, 1 H) 7.77 (d, *J*=9.1 Hz, 1 H) 8.00 (d, *J*=1.8 Hz, 1 H) 9.63 (s, 1 H) 10.68 (s, 1 H)

### Example 6:

8-chloro-N-(3-(2-(dimethylamino)ethyl)-1H-indol-5-yl)naphthalene-1-sulfonamide 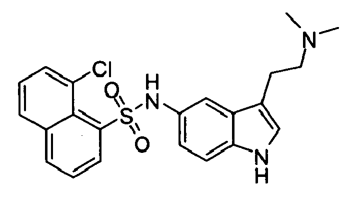 Melting point: 177-179°C

1 H NMR (300 MHz, DMSO-*d*₆) δ ppm 2.09 (s, 6 H) 2.30 (m, 2 H) 2.61 (m, 2 H) 6.91 (dd, *J*=8.64, 1.90 Hz, 1 H) 7.06 (d, *J*=1.90 Hz, 1 H) 7.14 - 7.20 (m, 2 H) 7.50 - 7.64 (m, 2 H) 7.86 (dd, *J*=7.5, 1.1 Hz, 1 H) 8.04 (d, *J*=7.2 Hz, 1 H) 8.18 (d, *J*=7.6 Hz, 1 H) 8.32 (m, 1 H) 10.02 (br. s., 1 H) 10.71 (br. s., 1 H)

### Example 7:

N-(3-(2-(dimethylamino)ethyl)-1H-indol-5-yl)-4-fluoronaphthalene-1-sulfonamide 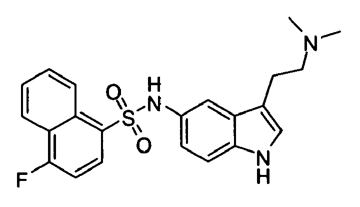 Melting point: 89-92°C

1H NMR (300 MHz, DMSO-*d*₆) δ ppm 2.09 (s, 6 H) 2.20 (m, 2 H) 2.54 (m, 2 H) 6.68 (dd, *J*=8.6, 1.7 Hz, 1 H) 6.88 (s, 1 H) 7.03 (s, 1 H) 7.08 (d, *J*=8.6 Hz, 1 H) 7.33 (m, 1 H) 7.79 (m, 2 H) 8.00 (m, 1 H) 8.15 (d, *J*=8.2 Hz, 1 H) 8.81 (d, *J*=8.6 Hz, 1 H) 10.10 (br. s., 1 H) 10.70 (br. s., 1H)

### Example 8:

5-chloro-N-(3-(2-(dimethylamino)ethyl)-1-methyl-1H-indol-5-yl)naphthalene-2-sulfonamide 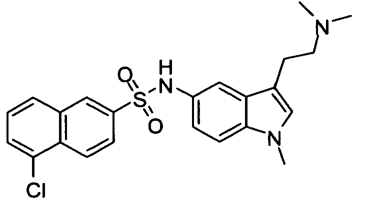 Melting point: 162-164°C

1H NMR (400 MHz, DMSO-*d*₆) δ ppm 1.98 (s, 6 H) 2.19 (m, 2 H) 2.55 (m, 2 H) 3.25 (s, 3 H) 6.80 (dd, *J*=8.6, 2.0 Hz, 1 H) 6.96 (d, *J*=2.0 Hz, 1 H) 7.14 (d, *J*=2.3 Hz, 1 H) 7.24 (d, *J*=8.6 Hz, 1 H) 7.60 - 7.70 (m, 2 H) 7.89 (d, *J*=6.6 Hz, 1 H) 8.15 (d, *J*=8.6 Hz, 1 H) 8.30 (d, *J*=9.0 Hz, 1 H) 8.36 (d, *J*=1.56 Hz, 1 H) 10.89 (s, 1 H)

### Example 9:

N-(1-methyl-3-(2-(pyrrolidin-1-yl)ethyl)-1H-indol-5-yl)naphthalene-1-sulfonamide 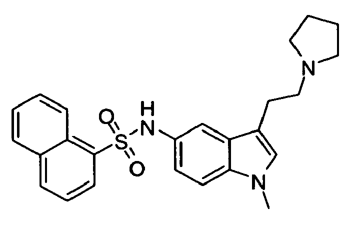 Melting point: 196-198°C

1 H NMR (300 MHz, DMSO-*d*₆) δ ppm 1.65 (m, 4 H) 2.30 - 2.39 (m, 6 H) 2.52 - 2.59 (m, 2 H) 3.56 (s, 3 H) 6.74 (dd, *J*=8.6, 2.0 Hz, 1 H) 6.93 (d, *J*=2.0 Hz, 1 H) 7.00 (s, 1 H) 7.11 (d, J=8.6 Hz, 1 H) 7.48 (dd, *J*=8.1, 7.5 Hz, 1 H) 7.64 (m, 1 H) 7.72 (m, 1 H) 7.96 - 8.06 (m, 2 H) 8.12 (d, *J*=8.2 Hz, 1 H) 8.78 (d, *J*=8.6 Hz, 1 H) 10.15 (br. s., 1 H)
N-(1-methyl-3-(2-(pyrrolidin-1-yl)ethyl)-1H-indol-5-yl)naphthalene-1-sulfonamide oxalate
Melting point: 190°C-desc

### Example 10:

N-(3-(2-(dimethylamino)ethyl)-1H-indol-5-yl)-N-methylnaphthalene-1-sulfonamide 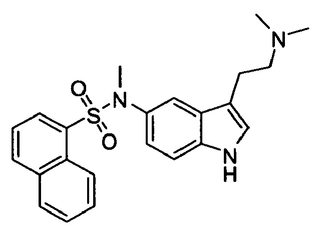 Melting point: 63-65°C

1 H NMR (300 MHz, DMSO-*d*₆) δ ppm 2.08 (s, 6 H) 2.18 (m, 2 H) 2.53 (m, 2 H) 3.24 (s, 3 H) 6.81 (dd, *J*=8.6, 2.0 Hz, 1 H) 6.92 (d, *J*=2.0 Hz, 1 H) 7.12 (d, *J*=2.3 Hz, 1 H) 7.20 (d, *J*=8.6 Hz, 1 H) 7.43 (m, 1 H) 7.55 - 7.62 (m, 2 H) 7.99 (dd, *J*=7.5, 1.2 Hz, 1 H) 8.06 (d, *J*=8.6 Hz, 1 H) 8.20 - 8.31 (m, 2 H) 10.87 (s, 1 H)
N-(3-(2-(dimethylamino)ethyl)-1H-indol-5-yl)-N-methylnaphthalene-1-sulfonamide oxalate
Melting point: 151-154°C

### Example 11:

N-(3-(2-(dimethylamino)ethyl)-1H-indol-5-yl)-N-methylnaphthalene-2-sulfonamide 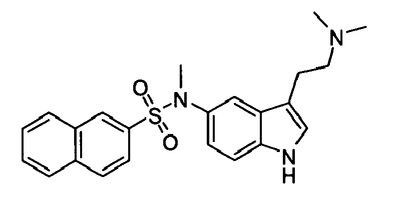 Melting point: 154-158°C

1 H NMR (300 MHz, DMSO-*d*₆) δ ppm 1.99 (s, 6 H) 2.22 (m, 2 H) 2.54 (m, 2 H) 3.23 (s, 3 H) 6.77 (dd, *J*=8.6, 2.0 Hz, 1 H) 6.99 (d, *J*=2.0 Hz, 1 H) 7.14 (d, *J*=2.2 Hz, 1 H) 7.23 (d, *J*=8.6 Hz, 1 H) 7.49 (dd, *J*=8.6, 1.8 Hz, 1 H) 7.60 - 7.74 (m, 2 H) 8.00 - 8.09 (m, 2 H) 8.11 (m, 1 H) 8.25 (d, *J*=1.7 Hz, 1 H) 10.90 (s, 1 H)

### N-(3-(2-(dimethylamino)ethyl)-1H-indol-5-yl)-N-methylnaphthalene-2-sulfonamide oxalate

Melting point: 186-189°C

### Example 12:

5-chloro-N-(3-(2-(dimethylamino)ethyl)-1H-indol-5-yl)-N-methylnaphthalene-1-sulfonamide 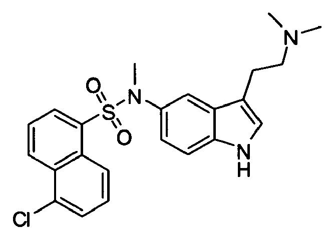 Melting point: 69-73°C

1 H NMR (300 MHz, DMSO-*d*₆) δ ppm 2.07 (s, 6 H) 2.14 (m, 2 H) 2.52 (m, 2 H) 3.25 (s, 3 H) 6.83 (dd, *J*=8.5, 2.1 Hz, 1 H) 6.87 (d, *J*=2.1 Hz, 1 H) 7.12 (d, *J*=2.3 Hz, 1 H) 7.22 (d, *J*=8.5 Hz, 1 H) 7.36 (dd, *J*=8.8, 7.4 Hz, 1 H) 7.73 - 7.82 (m, 2 H) 8.12 (dd, J=7.3, 1.2 Hz, 1 H) 8.23 (d, *J*=8.9 Hz, 1 H) 8.53 (d, *J*=8.5 Hz, 1 H) 10.88 (br. s., 1 H)
5-chloro-N-(3-(2-(dimethylamino)ethyl)-1H-indol-5-yl)-N-methylnaphthalene-1-sulfonamide oxalate
Melting point: 156-158°C

### Example 13:

5-chloro-N-(3-(2-(dimethylamino)ethyl)-1H-indol-5-yl)-N-methylnaphthalene-2-sulfonamide 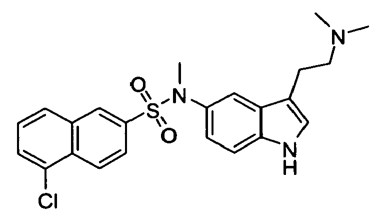 Melting point: 185-190°C

1H NMR (300 MHz, DMSO-*d*₆) δ ppm 2.02 (s, 6 H) 2.24 (m, 2 H) 2.57 (m, 2 H) 3.25 (s, 3 H) 6.79 (dd, *J*=8.6, 2.0 Hz, 1 H) 6.98 (s, 1 H) 7.15 (d, *J*=2.2 Hz, 1 H) 7.24 (d, *J*=8.6 Hz, 1 H) 7.61 - 7.69 (m, 2 H) 7.89 (d, *J*=7.5 Hz, 1 H) 8.16 (d, *J*=8.4 Hz, 1 H) 8.30 (d, *J*=8.9 Hz, 1 H) 8.36 (d, *J*=1.8 Hz, 1 H) 10.91 (br. s., 1 H)
5-chloro-N-(3-(2-(dimethylamino)ethyl)-1H-indol-5-yl)-N-methylnaphthalene-2-sulfonamide oxalate
Melting point: 114-116°C

### Example A:

### 5-methoxy-naphthalene-1-sulfonyl chloride

Sodium 5-hydroxy-1-napthalenesulfonate (26,5 g, 100 mmol) was suspended in water (250 mL), and NaOH (30 g) was added with stirring. To the solution was added dropwise dimethyl sulphate (64 ml) during 1h, maintaining the temperature below 60°C and the mixture was stirred at 55°C for 16 h. The dark solution was cooled, NaCl (50 g) was added and the precipitate was filtered off, washed with concentrated NaCl solution and toluene and dried overnight at 80°C affording 23,8 g of a cream-colored solid. This solid was suspended in dry dimethylformamide (50 ml) and cooled. Thionyl chloride (11,4ml) was added keeping the internal temperature below 15°C. The mixture was stirred 3h at room temperature, cooled, and treated with ice/water (300 ml) to obtain a yellowish suspension. The solid was filtered and dissolved in 800 ml of CH₂Cl₂. This solution was dried (Na₂SO₄) and evaporated to afford 18,4 g of 5-methoxy-naphthalene-1-sulfonyl chloride as an ochre-colored solid.

### Example 14:

N-(3-(2-(dimethylamino)ethyl)-1H-indol-5-yl)-5-methoxynanhthalene-1-sulfonamide

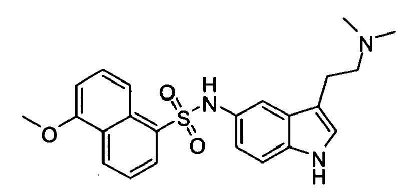

To a solution of 3-(2-(dimethylamino)ethyl)-1*H*-indoi-5-amine (14,5 g, 71,3 mmol) in 200 ml of pyridine was added dropwise, at -15°C, a solution of 5-methoxy-naphthalene-1-sulfonyl chloride (18,3, 71,3 mmol) in 50 ml of pyridine. The reaction mixture was stirred at room temperature overnight and then it was cooled, alkalinized with NaHCO₃ and NH₄OH and evaporated to dryness and the resulting solid was repeatedly washed with water/acetone (10:1) to yield 28,5 g of a solid that was crystallized from acetone/water 9:1 obtaining 22,1 g (73,2%) of N-(3-(2-(dimethylamino)ethyl)-1*H*-indol-5-yl)-5-methoxynaphthalene-1-sulfonamide as an ochre-colored solid
Melting point: 130°C-desc

1 H NMR (300 MHz, DMSO-*d*₆) δ ppm 2.08 (s, 6 H) 2.17 (m, 2 H) 2.51 (m, 2 H) 3.96 (s, 3 H) 6.68 (dd, *J*=8.6, 2.0 Hz, 1 H) 6.89 (d, *J*=1.9 Hz, 1 H) 7.01 (d, *J*=2.2 Hz, 1 H) 7.05 (d, *J*=8.6 Hz, 1 H) 7.10 (d, *J*=7.9 Hz, 1 H) 7.45 (dd, *J*=8.4, 7.4 Hz, 1 H) 7.62 (dd, *J*=8.8, 7.9 Hz, 1 H) 8.01 (dd, *J*=7.4, 1.4 Hz, 1 H) 8.29 - 8.39 (m, 2 H) 10.04 (br. s., 1 H) 10.66 (s, 1 H)

### Example 15 (1^{st} route):

N-(3-(2-(dimethylamino)ethyl)-1*H*-indol-5-yl)-5-hydroxynaphthalene-1-sulfonamide

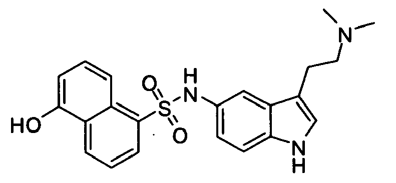

To a suspension of N-(3-(2-(dimethylamino)ethyl)-1*H*-indol-5-yl)-5-methoxynaphthalene-1-sulfonamide (12,5g 29,5 mmol) in 600 ml of CH₂Cl₂, BBr₃(1.0M in CH₂Cl₂, 88,5 ml, 88,5 mmol) was added dropwise at -78°C and then the mixture was stirred at room temperature overnight (14 h). The reaction was poured over ice-water and basified with NaHCO₃. The resulting tan solid was separate from liquid phases by decantation, dissolved in 250 ml of MeOH, basified with NH₄OH dil. and the solution was added dropwise over 2000 ml of water. The resultant precipitate was filtered, washed with water and dried to afford 8,6 g (72% yield) of N-(3-(2-(dimethylamino)ethyl)-1H-indol-5-yl)-5-hydroxynaphthalene-1-sulfonamide as an ochre-colored solid
Melting point: 210-12°C

1H NMR (300 MHz, DMSO-*d*₆) δ ppm 2.10 (s, 6 H) 2.21 (m, 2 H) 2.54 (m, 2 H) 6.68 (dd, *J*=8.5, 1.9 Hz, 1 H) 6.93 (d, *J*=1.9 Hz, 1 H) 6.97 (d, *J*=7.2 Hz, 1 H) 7.01 (d, *J*=2.2 Hz, 1 H) 7.05 (d, *J*=8.5 Hz, 1 H) 7.38 (m, 1 H) 7.49 (m, 1 H) 7.98 (dd, *J*=7.3, 1.2 Hz, 1 H) 8.20 (d, *J*=8.8 Hz, 1 H) 8.33 (d, *J*=8.4 Hz, 1 H) 10.02 (br. s., 1 H) 10.66 (s, 1 H)

### Example B:

### 5-(chlorosulfonyl)naphthalen-1-yl 4-methylbenzenesulfonate

p-Toluenesulfonyl chloride (23,6 g, 124 mmol) was added at room temperature to a mixture of sodium 5-hydroxy-1-napthalenesulfonate ( 33,0 g, 124 mmol) and 32 ml of NaOH 5N in 32 ml of H₂O and 32 ml of EtOH. The mixture was stirred at 100°C for 3h. Then it was cooled, NaCl (50g) was added and was left to precipitate overnight at room temperature. Then it was cooled, filtered and the solid washed with cold water and toluene and dried at 60°C in vacuum. The solid was stirred with phosphorous pentachloride (52 g) at 70°C during 2 h. It was cooled, ice-water (100 ml) was added and extracted with CH₂Cl₂. The organic layer was separated, dried (Na₂SO₄) and evaporated to dryness to afford 33,6 g of a solid of 67% purity (LC) that was used without further purification.

### Example 15 (2^{nd} route):

N-(3-(2-(dimethylamino)ethyl)-1*H*-indol-5-yl)-5-hydroxynaphthalene-1-sulfonamide

To a solution of 3-(2-(dimethylamino)ethyl)-1*H*-indol-5-amine (11,2 g, 55 mmol) in 500 ml of pyridine was added dropwise, at -15°C, a solution of 5-(chlorosulfonyl)naphthalen-1-yl 4-methylbenzenesulfonate (55,0 mmol) in 50 ml of pyridine. The reaction mixture was stirred at room temperature overnight and then it was cooled, alkalinized with NaHCO₃ (20 ml sat.sol.)and NH₄OH (5 ml) and evaporated to dryness. The resulting residue was taken with water and extracted with CHCl₃. The organic layer was separated, dried (Na₂SO₄) and evaporated to dryness to obtain a mass of 5-(N-(3-(2-(dimethylamino)ethyl)-1H-indol-5-yl)sulfamoyl)naphthalen-1-yl 4-methylbenzenesulfonate that was refluxed with NaOH 5N (80 ml) in 600 ml of MeOH. Then, methanol was evaporated and the aqueous solution was acidified with HCl dil. to obtain a brown solid that was purified by flash chromatography (silicagel) with CH₂Cl₂/MeOH/NH₄OH (6:4:0,02) yielding 8,3 g (37%) of N-(3-(2-(dimethylamino)ethyl)-1*H*-indol-5-yl)-5-hydroxynaphthalene-1-sulfonamide as a solid

The following compounds may be prepared by a similar procedure (to examples 14 and 15) from the corresponding naphthalene:

### Example 16:

N-(3-(2-(dimethylamino)ethyl)-1H-indol-5-yl)-7-methoxynaphthalene-2-sulfonamide 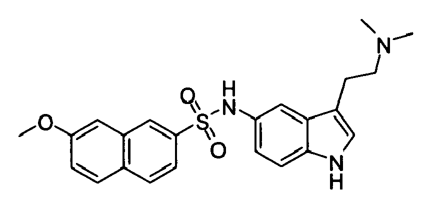 Melting point: 95°C-desc

1 H NMR (300 MHz, DMSO-*d*₆) δ ppm 2.10 (s, 6 H) 2.31 (m, 2 H) 2.61 (m, 2 H) 3.83 (s, 3 H) 6.79 (dd, *J*=8.6, 2.0 Hz, 1 H) 7.05 (d, *J*=2.0 Hz, 1 H) 7.09 (d, *J*=1.8 Hz, 1 H) 7.12 (d, *J*=8.6 Hz, 1 H) 7.26 (dd, *J*=9.0, 2.5 Hz, 1 H) 7.44 (d, *J*=2.5 Hz, 1 H) 7.59 (dd, *J*=8.6, 1.7 Hz, 1 H) 7.86 (d, *J*=9.0 Hz, 1 H) 7.95 (d, *J*=8.6 Hz, 1 H) 8.15 (d, *J*=1.7 Hz, 1 H) 9.82 (br. s., 1 H) 10.73 (br. s., 1 H)

### Example 17:

N-(3-(2-(dimethylamino)ethyl)-1 H-indol-5-yl)-7-hydroxynaphthalene-2-sulfonamide 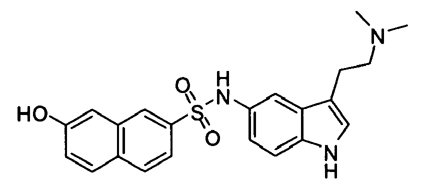 Melting point: 138-145°C

1 H NMR (400 MHz, DMSO-*d*₆) δ ppm 2.33 (s., 6 H) 2.62 (m, 2 H) 2.73 (m, 2 H) 6.76 (dd, *J*=8.6, 2,0 Hz, 1 H) 7.10 (d, *J*=2,0 Hz, 1 H) 7.12 (d, *J*=8.6 Hz, 1 H) 7.16 - 7.22 (m, 3 H) 7.49 (dd, *J*=8.6, 2,0 Hz, 1 H) 7.80 (d, *J*=8.6 Hz, 1 H) 7.87 (d, *J*=8.6 Hz, 1 H) 8.02 (s, 1 H) 9.80 (br. s., 1 H) 10.03 (m, 1H) 10.78 (s, 1 H)

### Example 18:

N-(3-(2-(dimethylamino)ethyl)-1H-indol-5-yl)-8-methoxynaphthalene-1-sulfonamide 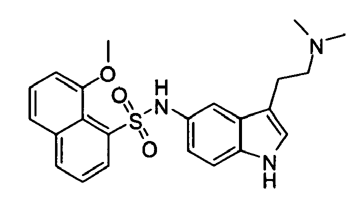 Melting point: 89-92°C

1 H NMR (300 MHz, DMSO-*d*₆) δ ppm 2.05 (s, 6 H) 2.21 (m, 2 H) 2.56 (m, 2 H) 4.11 (s, 3H) 6.79 (dd, *J*=8.6, 1.9 Hz, 1 H) 7.04 (d, *J*=2.2 Hz, 1 H) 7.06 (d, J=1.9 Hz, 1 H) 7.10 (d, J=8.6 Hz, 1 H) 7.35 (dd, *J*=7.3, 1.8 Hz, 1 H) 7.43 (t, J=7.9 Hz, 1 H) 7.55 - 7.67 (m, 2 H) 8.03 - 8.11 (m, 2 H) 8.72 (s, 1 H) 10.70 (m, 1 H)

### Example 19:

N-(3-(2-(dimethylamino)ethyl)-1H-indol-5-yl)-8-hydroxynaphthalene-1-sulfonamide 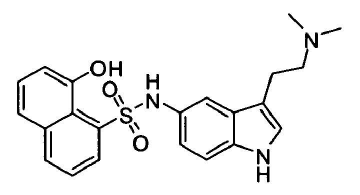 Melting point: 164-165°C
1 H NMR (400 MHz, DMSO-*d*₆) δ ppm 2.36 (s, 6 H) 2.62 (m, 2 H) 2.71 (m, 2 H) 6.74 (dd, *J*=8.6, 2,0 Hz, 1 H) 7.08 - 7.11 (m, 2 H) 7.16 - 7.20 (m, 2 H) 7.36 (t, *J*=7.8 Hz, 1 H) 7.44 - 7.52 (m, 2 H) 7.98 (m, 1 H) 8.01 (m, 1 H) 10.76 (s, 1 H)

### Example 20:

N-(3-(2-(dimethylamino)ethyl)-1H-indol-5-yl)-4-methoxynaphthalene-1-sulfonamide 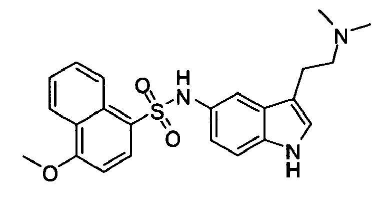 Melting point: 165-167°C

1 H NMR (300 MHz, DMSO-*d*₆) δ ppm 2.09 (s, 6 H) 2.21 (m, 2 H) 2.54 (m, 2 H) 3.95 (s, 3 H) 6.69 (dd, *J*=8.6, 2.1 Hz, 1 H) 6.90 - 6.95 (m, 2 H) 7.01 (d, *J*=2.2 Hz, 1 H) 7.05 (d, *J*=8.6 Hz, 1 H) 7.61 (m, 1 H) 7.72 (m, 1 H) 7.96 (d, *J*=8.4 Hz, 1 H) 8.22 (d, *J*=8.4, Hz, 1 H) 8.73 (d, *J*=8.6 Hz, 1 H) 9.94 (br. s., 1 H) 10.66 (s, 1 H)

### Example 21:

(+)-N-(3-((1-methylpyrrolidin-2-yl)methyl)-1H-indol-5-yl)naphthalene-2-sulfonamide 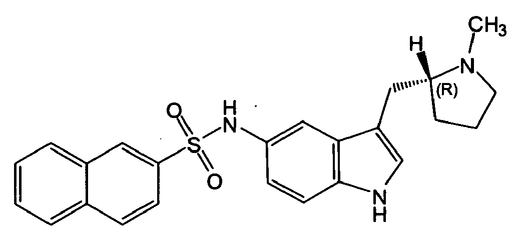 Melting point: 114-116°C
[α]²⁵_{D(C=0.3,MeOH)} +46_{.}2°

1 H NMR (300 MHz, DMSO-*d*₆) δ ppm 1.09-1.48 (m, 4 H) 1.84 - 2.00 (m, 2 H) 2.13 (s, 3 H) 2.23 (m, 1 H) 2.75 (m, 1 H) 2.83 (m, 1 H) 6.83 (dd, *J*=8.6, 2.0 Hz, 1 H) 7.00-7.04 (m, 2H) 7.13 (d, *J*=8.6 Hz, 1 H) 7.54-7.67 (m, 2 H) 7.73 (dd, J=8.6, 1.8 Hz, 1 H) 7.93-8.06 (m, 3 H) 8.23 (m, 1 H) 9.82 (br. s., 1 H) 10.72 (s, 1 H)

(+)-N-(3-((1-methylpyrrolidin-2-yl)methyl)-1H-indol-5-yl)naphthalene-2-sulfonamide oxalate
Melting point: 175°C desc
[α]²⁵_{D (C=0.3,MeOH)} +21,0°

### Example 22:

(-)-N-(3-((1-methylpyrrolidin-2-yl)methyl)-1H-indol-5-yl)naphthalene-2-sulfonamide 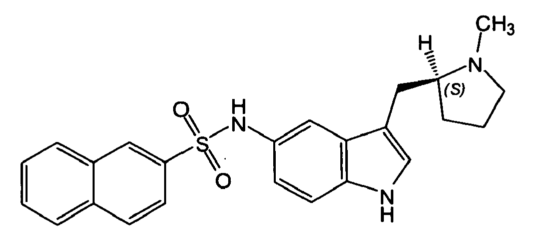 Melting point: 112-115°C
[α]²⁵_{D(C=0.3,MeOH)} -46.2°

1 H NMR (300 MHz, DMSO-*d*₆) δ ppm 1.09-1.48 (m, 4 H) 1.84 - 2.00 (m, 2 H) 2.13 (s, 3 H) 2.23 (m, 1 H) 2.75 (m, 1 H) 2.83 (m, 1 H) 6.83 (dd, *J*=8.6, 2.0 Hz, 1 H) 7.00-7.04 (m, 2H) 7.13 (d, *J*=8.6 Hz, 1 H) 7.54-7.67 (m, 2 H) 7.73 (dd, *J*=8.6, 1.8 Hz, 1 H) 7.93-8.06 (m, 3 H) 8.23 (m, 1 H) 9.82 (br. s., 1 H) 10.72 (s, 1 H)

(-)-N-(3-((1-methylpyrrolidin-2-yl)methyl)-1H-indol-5-yl)naphthalene-2-sulfonamide oxalate
[α]²⁵_{D(C=0.3,MeOH)} -21.0°

### BIOLOGICAL ASSAYS

### BINDING WITH SEROTONIN RECEPTOR 5HT₆

Cell membranes of HEK-293 cells expressing the 5HT₆ human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytriptamine-7 Receptors. The Journal of Pharmacology and Experimental Therapeutics, 1994, 268, 1403] with slight changes. The commercial membrane is diluted (1:40 dilution) with the binding buffer. 50 mM Tris-HCl, 10 mM MgCl₂, 0.5 mM EDTA (pH 7.4). The radioligand used is [³H]-LSD at a concentration of 2.7 nM with a final volume of 200 µl. incubation is initiated by adding 100 µl of membrane suspension, (≈ 22.9 µg membrane protein), and is prolonged for 60 minutes at a temperature of 37°C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Nonspecific binding is determined in the presence of 100 µM of serotonin. Tests were made in triplicate. The inhibition constants (Kᵢ, nM) were calculated by non-linear regression analysis using the program EBDA/LIGAND [Munson and Rodbard, Analytical Biochemistry, 1980, 107, 220].
The following table shows results indicative of binding for some of the compounds object of the present invention.

| **Table** | | | |
|---|---|---|---|
| **Example** | **Binding % Inhibition 100 nM** | **Binding % Inhibition 10 nM** | **Binding 5HT₆ Kᵢ (nM)** |
| 1 | 84.4 | 73.9 | 4.4 ± 1.1 |
| 2 | 86.3 | 75.1 | 5.3 |
| 3 | 88.5 | 80.3 | 9.8 |
| 4 | 89.3 | 81.2 | 3.9±0.4 |
| 5 | 88.5 | 69.8 | 11.9 ± 3.3 |
| 6 | 88.4 | 68.4 | 2.1 |
| 7 | 95.4 | 81.5 | 5.2 |
| 8 | 93.4 | 64.5 | 13.4 ± 0.9 |
| 9 | 86.8 | 58.1 | 42.5 ± 13.9 |
| 10 | 92.7 | 79.2 | 9.7 ± 4.5 |
| 11 | 93.1 | 73.1 | 12.3 ± 3.7 |
| 12 | 85.4 | 51.6 | 48.7 ± 6.9 |
| 13 | 87.6 | 55.5 | 18.1 ± 2.6 |
| 14 | 88.1 | 59.6 | 18.6 |
| 15 | 94.6 | 91.9 | 0.50 |
| 16 | 89.3 | 70.0 | 5.0 ± 1.3 |
| 17 | 91.5 | 69.9 | 6.9 ± 0.2 |
| 18 | 88.4 | 69.2 | 8.3 |
| 19 | 85.9 | 53.8 | 24.6 ± 2.1 |
| 20 | 81.8 | 53.3 | 8.5 ± 1.7 |

The daily doses in human medicine are between 1 milligram and 500 milligrams of product, which can be given in one or more administrations. The compositions are prepared in forms compatible with the administration means used, such as sugar-coated pills, tablets, capsules, suppositories, solutions or suspensions. These compositions are prepared by known methods and comprise between 1 and 60% by weight of the active principle (compound with the general formula I) and 40 to 99% by weight of a suitable pharmaceutical vehicle compatible with the active principle and the physical form of the composition used. By way of example, the formula of a tablet containing a product of the invention is shown.

**Example of formula per tablet:**

| | |
|---|---|
| Example 15 | 5 mg |
| Lactose | 60 mg |
| Crystalline cellulose | 25 mg |
| K 90 Povidone | 5 mg |
| Pregelatinised starch | 3 mg |
| Colloidal silicon dioxide | 1 mg |
| Magnesium stearate | 1 mg |
| Total weight per tablet | 100 mg |

## Claims

1. A naphthyl-substituted sulfonamide compound according to general formula (I) 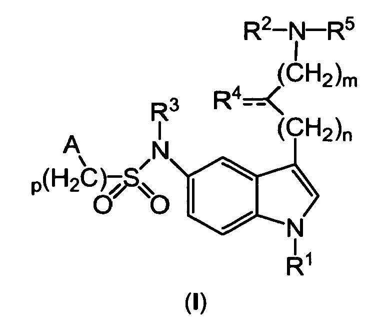 wherein
**A** represents a naphthyl radical, unsubstituted, or mono- or di-substituted with any of U, **V** or **W** selected from halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl or a benzyl radical;
**R²** represents hydrogen, C₁-C₄ alkyl; optionally at least monosubstituted alkyl-aryl; or C(O)-R⁶ with R⁶ being at least mono-substituted aryl;
**R³** represents hydrogen or C₁-C₄ alkyl;
**R⁴** represents hydrogen or C₁-C₄ alkyl;
**or**
**R²** and **R⁴** together with the connecting atoms are forming an optionally monosubstituted, optionally monounsaturated 5- or 6-membered heterocyclic ring; **R⁵** represents hydrogen or C₁-C₄ alkyl
or
**R²** and **R⁵** together with the connecting nitrogen are forming an optionally monosubstituted 5- or 6-membered heterocyclic ring, optionally condensed with an un-substituted phenyl;
**n** represents 0, or 1;
**m** represents 0, 1 or 2;
**p** represents 0, 1 or 2;
^{....} represents either a single or a double bond;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

2. An naphthyl-substituted sulfonamide compound according to claim 1, wherein the compounds are compounds according to formula (Ia) 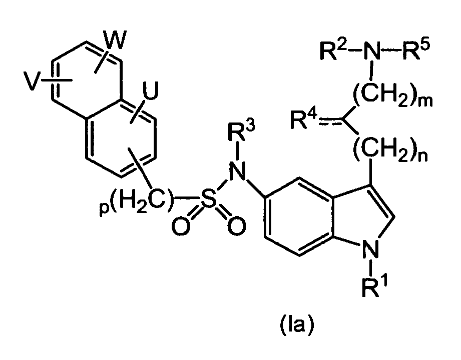 wherein
**U, V** and **W** independently from one another represent hydrogen; halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl or a benzyl radical;
**R²** represents hydrogen, C₁-C₄ alkyl; optionally at least monosubstituted alkyl-aryl; or C(O)-R⁶ with R⁶ being at least mono-substituted aryl;
**R³** represents hydrogen or C₁-C₄ alkyl;
**R⁴** represents hydrogen or C₁-C₄ alkyl;
**or**
**R²** and **R⁴** together with the connecting atoms are forming an optionally monosubstituted, optionally monounsaturated 5- or 6-membered heterocyclic ring; **R⁵** represents hydrogen or C₁-C₄ alkyl
**or**
**R²** and **R⁵** together with the connecting nitrogen are forming an optionally monosubstituted 5- or 6-membered heterocyclic ring, optionally condensed with an un-substituted phenyl;
**n** represents 0, or 1;
**m** represents 0, 1 or 2.

3. A naphthyl-substituted sulfonamide compound according to claim 1, wherein the compounds are compounds according to general formula (II) 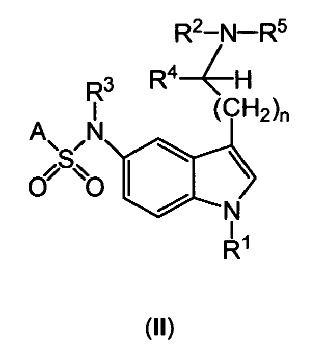 wherein
**A** represents a naphthyl radical, unsubstituted, or mono- or di-substituted with any of U, **V** or **W** selected from halogen; C₁-C₄ alkyl; or NR⁶R⁷ or OR⁶ with **R⁶** and **R⁷** independently from one another being selected from H or C₁-C₄ alkyl;
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
**or**
**R²** and **R⁴** together with the connecting atoms are forming an optionally monosubstituted, optionally monounsaturated 5- or 6-membered heterocyclic ring; **R⁵** represents hydrogen or C₁-C₄ alkyl
**or**
**R²** and **R⁵** together with the connecting nitrogen are forming an optionally monosubstituted 5- or 6-membered heterocyclic ring;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

4. A naphthyl-substituted sulfonamide compound according to claim 3, wherein the compounds are compounds according to general formula (IIa) 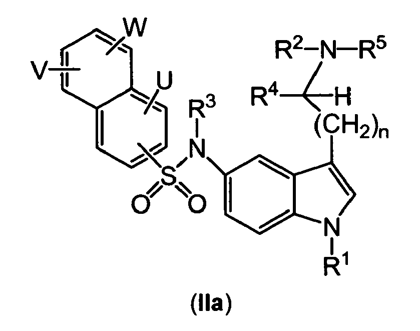 wherein
**U, V** and **W** independently from one another represent hydrogen; halogen; C₁-C₄ alkyl; or NR⁶R⁷ or OR⁶ with R⁶ and R⁷ independently from one another being selected from H or C₁-C₄ alkyl;
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
or
**R²** and **R⁴** together with the connecting atoms are forming an optionally monosubstituted, optionally monounsaturated 5- or 6-membered heterocyclic ring; **R⁵** represents hydrogen or C₁-C₄ alkyl
or
**R²** and **R⁵** together with the connecting nitrogen are forming an optionally monosubstituted 5- or 6-membered heterocyclic ring;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

5. A naphthyl-substituted sulfonamide compound according to claim 4, wherein the compounds are compounds according to general formula (IIb) 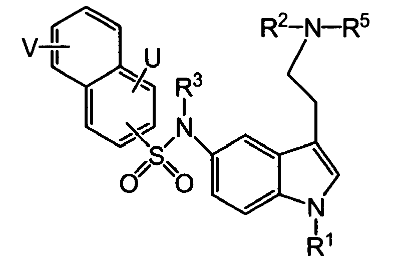 , wherein
one of **U** and **V** represents hydrogen; halogen; C₁-C₄ alkyl; or NR⁶R⁷ or OR⁶ with R⁶ and R⁷ independently from one another being selected from H or C₁-C₄ alkyl, while the other represents hydrogen;
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
or
**R²** and **R⁵** together with the connecting nitrogen are forming an unsubstituted pyrrolidine.

6. A naphthyl-substituted sulfonamide compound according to any of claims 1 to 5,
wherein
one of **U** and **V** represents hydrogen; F, Cl; methyl; or N(CH₃)₂ or OR⁶ with R⁶ being selected from H or C₁-C₄ alkyl, while the other represents hydrogen;
**R¹** represents hydrogen, methyl or ethyl; preferably represents hydrogen or methyl; **and/or**
**R³** represents hydrogen, methyl, or ethyl; preferably represents hydrogen or methyl; **and/or**
**R²** represents hydrogen, methyl, ethyl or propyl; preferably represents methyl;
**R⁵** represents hydrogen, methyl or ethyl; preferably represents methyl;
**or**
**R²** and **R⁵** together with the connecting nitrogen are forming an unsubstituted pyrrolidine.

7. A naphthyl-substituted sulfonamide compound according to any of claim 3 to 6, wherein the compounds are selected from
• 6-chloro-N-(3-(2-(dimethylamino)ethyl)-1H-indol-5-yl)naphthalene-2-sulfonamide;
• 5-(dimethylamino)-N-(3-(2-(dimethylamino)ethyl)-1H-indol-5-yl)naphthalene-1-sulfonamide;
• N-(3-(2-(dimethylamino)ethyl)-1H-indol-5-yl)-4-methylnaphthalene-1-sulfonamide;
• 7-chloro-N-(3-(2-(dimethylamino)ethyl)-1H-indol-5-yl)naphthalene-2-sulfonamide;
• 6-(dimethylamino)-N-(3-(2-(dimethylamino)ethyl)-1H-indol-5-yl)naphthalene-2-sulfonamide;
• 8-chloro-N-(3-(2-(dimethylamino)ethyl)-1H-indol-5-yl)naphthalene-1-sulfonamide;
• N-(3-(2-(dimethylamino)ethyl)-1H-indol-5-yl)-4-fluoronaphthalene-1-sulfonamide
• 5-chloro-N-(3-(2-(dimethylamino)ethyl)-1-methyl-1H-indol-5-yl)naphthalene-2-sulfonamide;
• N-(1-methyl-3-(2-(pyrrolidin-1-yl)ethyl)-1H-indol-5-yl)naphthalene-1-sulfonamide
• N-(3-(2-(dimethylamino)ethyl)-1H-indol-5-yl)-N-methylnaphthalene-1-sulfonamide;
• N-(3-(2-(dimethylamino)ethyl)-1H-indol-5-yl)-N-methylnaphthalene-2-sulfonamide;
• 5-chloro-N-(3-(2-(dimethylamino)ethyl)-1H-indol-5-yl)-N-methylnaphthalene-1-sulfonamide;
• 5-chloro-N-(3-(2-(dimethylamino)ethyl)-1H-indol-5-yl)-N-methylnaphthalene-2-sulfonamide;
• N-(3-(2-(dimethylamino)ethyl)-1H-indol-5-yl)-5-methoxynaphthalene-1-sulfonamide;
• N-(3-(2-(dimethylamino)ethyl)-1H-indol-5-yl)-5-hydroxynaphthalene-1-sulfonamide;
• N-(3-(2-(dimethylamino)ethyl)-1H-indol-5-yl)-7-methoxynaphthalene-2-sulfonamide;
• N-(3-(2-(dimethylamino)ethyl)-1H-indol-5-yl)-7-hydroxynaphthalene-2-sulfonamide;
• N-(3-(2-(dimethylamino)ethyl)-1H-indol-5-yl)-8-methoxynaphthalene-1-sulfonamide;
• N-(3-(2-(dimethylamino)ethyl)-1H-indol-5-yl)-8-hydroxynaphthalene-1-sulfonamide; or
• N-(3-(2-(dimethylamino)ethyl)-1H-indol-5-yl)-4-methoxynaphthalene-1-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

8. A naphthyl-substituted sulfonamide compound according to claim 1, wherein the compounds are compounds according to formula (III) 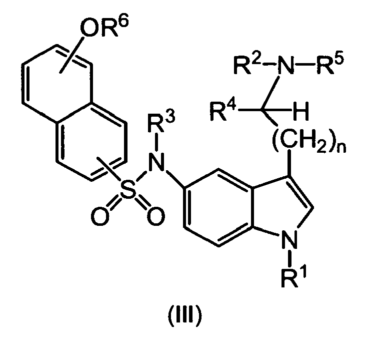 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
**or**
**R²** and **R⁴** together with the connecting atoms are forming an optionally monosubstituted, optionally monounsaturated 5- or 6-membered heterocyclic ring; **R⁵** represents hydrogen or C₁-C₄ alkyl
**or**
**R²** and **R⁵** together with the connecting nitrogen are forming an optionally monosubstituted 5- or 6-membered heterocyclic ring;
**R⁶** represents hydrogen or C₁-C₄ alkyl;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

9. A naphthyl-substituted sulfonamide compound according to claim 8, wherein the compounds are compounds according to formula (IIIa) 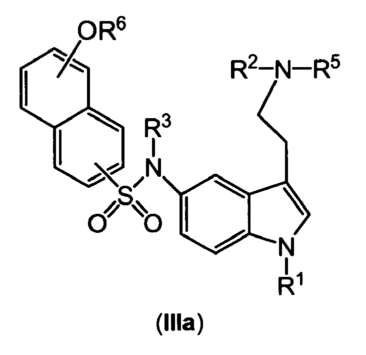 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
or
**R²** and **R⁵** together with the connecting nitrogen are forming an unsubstituted pyrrolidine;
**R⁶** represents hydrogen or C₁-C₄ alkyl.

10. A naphthyl-substituted sulfonamide compound according to any of claims 8 or 9,
wherein
**R¹** represents hydrogen, methyl or ethyl; preferably represents hydrogen or methyl; **and/or**
**R³** represents hydrogen, methyl, or ethyl; preferably represents hydrogen or methyl; **and/or**
**R⁶** represents hydrogen, methyl, or ethyl; preferably represents hydrogen or methyl; **and/or**
**R²** represents hydrogen, methyl, ethyl or propyl; preferably represents methyl;
**R⁵** represents hydrogen, methyl or ethyl; preferably represents methyl;
**or**
**R²** and **R⁵** together with the connecting nitrogen are forming an unsubstituted pyrrolidine.

11. A naphthyl-substituted sulfonamide compound according to claim 8, wherein the compounds are selected from
• N-(3-(2-(dimethylamino)ethyl)-1H-indol-5-yl)-5-methoxynaphthalene-1-sulfonamide;
• N-(3-(2-(dimethylamino)ethyl)-1H-indol-5-yl)-5-hydroxynaphthalene-1-sulfonamide;
• N-(3-(2-(dimethylamino)ethyl)-1H-indol-5-yl)-7-methoxynaphthalene-2-sulfonamide;
• N-(3-(2-(dimethylamino)ethyl)-1H-indol-5-yl)-7-hydroxynaphthalene-2-sulfonamide;
• N-(3-(2-(dimethylamino)ethyl)-1H-indol-5-yl)-8-methoxynaphthalene-1-sulfonamide;
• N-(3-(2-(dimethylamino)ethyl)-1H-indol-5-yl)-8-hydroxynaphthalene-1-sulfonamide; or
• N-(3-(2-(dimethylamino)ethyl)-1H-indol-5-yl)-4-methoxynaphthalene-1-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

12. A naphthyl-substituted sulfonamide compound according to claim 1, wherein the compounds are compounds according to formula (IV) 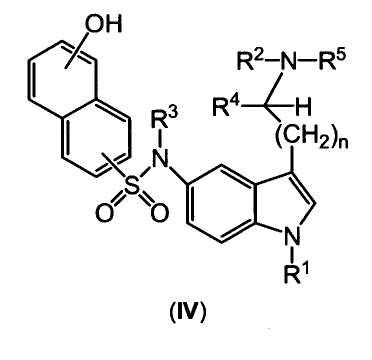 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁴** represents hydrogen;
**or**
**R²** and **R⁴** together with the connecting atoms are forming an optionally monosubstituted, optionally monounsaturated 5- or 6-membered heterocyclic ring; **R⁵** represents hydrogen or C₁-C₄ alkyl
**or**
**R²** and **R⁵** together with the connecting nitrogen are forming an optionally monosubstituted 5- or 6-membered heterocyclic ring;
**n** represents 0 or 1;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

13. A naphthyl-substituted sulfonamide compound according to claim 12, wherein the compounds are compounds according to formula (IVa) 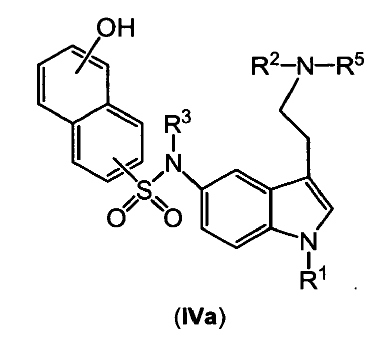 wherein
**R¹** represents hydrogen, or C₁-C₄ alkyl;
**R²** represents hydrogen, or C₁-C₄ alkyl;
**R³** represents hydrogen or C₁-C₄ alkyl;
**R⁵** represents hydrogen or C₁-C₄ alkyl
**or**
**R²** and **R⁵** together with the connecting nitrogen are forming an unsubstituted pyrrolidine.

14. A naphthyl-substituted sulfonamide compound according to any of claims 12 or 13, wherein
**R¹** represents hydrogen, methyl or ethyl; preferably represents hydrogen or methyl; **and/or**
**R³** represents hydrogen, methyl, or ethyl; preferably represents hydrogen or methyl; **and/or**
**R²** represents hydrogen, methyl, ethyl or propyl; preferably represents methyl;
**R⁵** represents hydrogen, methyl or ethyl; preferably represents methyl;
**or**
**R²** and **R⁵** together with the connecting nitrogen are forming an unsubstituted pyrrolidine.

15. A naphthyl-substituted sulfonamide compound according to claims 12 to 14, wherein the compounds are selected from
• N-(3-(2-(dimethylamino)ethyl)-1H-indol-5-yl)-5-hydroxynaphthalene-1-sulfonamide;
• N-(3-(2-(dimethylamino)ethyl)-1H-indol-5-yl)-7-hydroxynaphthalene-2-sulfonamide; or
• N-(3-(2-(dimethylamino)ethyl)-1H-indol-5-yl)-8-hydroxynaphthalene-1-sulfonamide;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

16. A medicament comprising an active substance according to one or more of claims 1 to 15 and optionally one or more pharmacologically acceptable adjuvants.

17. A medicament according to claim 16 for regulation of appetite, for maintenance, increase or reduction of body weight, for prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.

18. Use of a compound according to one or more of claims 1 to 15, for the manufacture of a medicament for the prophylaxis and/or treatment of disorders related to food ingestion, preferably for prophylaxis and/or treatment of obesity, anorexia, cachexia, bulimia, diabetes, preferably type II diabetes (non-insulin-dependent diabetes mellitus), or for prophylaxis and/or treatment of gastrointestinal tract disorders, preferably of the irritable bowel syndrome, for prophylaxis and/or treatment of Metabolic Syndrome, Peripheral Nervous System Disorders, Central Nervous System Disorders, arthritis, epilepsy, anxiety, panic, depression, cognitive disorders, memory disorders, cardiovascular diseases, senile dementia processes, such as Alzheimer's, Parkinson's and/or Huntington's Disease, schizophrenia, psychosis, infantile hyperkinesia (ADHD, attention deficit / hyperactivity disorder), pain, hypertensive syndrome, inflammatory diseases, immunologic diseases or for improvement of cognition.
